(19) Europäisches Patentamt
European Patent Office
Office européen des brevets

(11) **EP 1 648 824 B1**

(12) **EUROPEAN PATENT SPECIFICATION**

(45) Date of publication and mention
of the grant of the patent:
**12.12.2007   Bulletin 2007/50**

(51) Int Cl.:
**C01B 33/193** (2006.01)     **C01B 33/18** (2006.01)

(21) Application number: **04744085.4**

(86) International application number:
**PCT/IB2004/002431**

(22) Date of filing: **29.07.2004**

(87) International publication number:
**WO 2005/012175 (10.02.2005 Gazette 2005/06)**

(54) **AMORPHOUS SILICA PARTICLES HAVING HIGH ABSORBING CAPABILITIES AND HIGH STRUCTURAL CHARACTERISTICS**

AMORPHE KIESELSÄUREPARTIKEL MIT HOHER ABSORPTIONSKAPAZITÄT UND AUSGEPRÄGTEN STRUKTURELLEN EIGENSCHAFTEN

PARTICULES DE SILICE AMORPHE A HAUTE CAPACITE D'ABSORPTION ET A CARACTERISTIQUES STRUCTURALES DE HAUTE QUALITE

(84) Designated Contracting States:
**AT BE BG CH CY CZ DE DK EE ES FI FR GB GR HU IE IT LI LU MC NL PL PT RO SE SI SK TR**

(30) Priority: **01.08.2003   JP 2003285163**

(43) Date of publication of application:
**26.04.2006   Bulletin 2006/17**

(73) Proprietor: **DSL Japan Co. Ltd.**
**Shinjuku-ku, 163-0938 Tokyo (JP)**

(72) Inventors:
• **NISHI, Shugo**
**Amagasaki,**
**Hyogo 660-0813 (JP)**
• **TOKUNAGA, Tatsuya**
**Wake-gun,**
**Okayama 709-0441 (JP)**
• **OYAMA, Hisayo**
**Himeji,**
**Hyogo 670-0955 (JP)**

(74) Representative: **Lang, Arne**
**Degussa AG**
**Intellectual Property Management**
**Patente - Marken**
**Bau 1042 - PB 15**
**45764 Marl (DE)**

(56) References cited:
**WO-A-03/055802**          **US-A- 5 922 298**
**US-A1- 2002 102 198**

## Description

The present invention relates to amorphous silica particles having high absorbing capabilities and high structural characteristics. More particularly, the present invention relates to the amorphous silica particles, in which the maximum value of $\Delta Vp/\Delta Rp$ (where Vp is the pore volume [mm$^3$/g] and Rp is the pore radius [nm]) is 20 mm$^3$/nm·g$^{-1}$ or more in the pore distribution curve obtained by a benzene adsorption isotherm method, and the pore peak radius when the $\Delta Vp/\Delta Rp$ is maximum is from 20 nm or more to 100 nm or less. The present invention further relates to a process for manufacture of the amorphous silica of the invention as well as to the use of the silica of the invention.

Silica has been used for applications in a wide variety of fields, such as a reinforcing filler for rubber, a carrier for agrochemicals, a chemical absorbent, a filler for making paper, a coating agent for special paper, a resin compounding agent, a matting agent for coating material or the like, according to the physical and chemical characteristics of silica, and these required properties differ according to the application necessitating the marketing of many types of silica. In these applications, high oil absorbency is required for silica used in chemical absorbing (adsorbing and oil absorbing) agents, such as pharmaceuticals, agrochemicals, animal medicines, bathing agents or the like, a filler for making paper, a coating agent for special paper, a resin compounding agent, a matting agent for coating material or the like.

In general, the amorphous silica particles can be made by neutralizing an aqueous solution of alkali metal silicate and a mineral acid, and this fabrication method is called as a wet process. The wet process is classified into a precipitation method and a gel method. In the precipitation method, precipitated silicic acid, which is comparatively easily filtrated, is made by adding an acid to a sodium silicate aqueous solution, which is a base, and reacting it under neutral or alkaline. In the gel method, gelatinous silicic acid is made by adding a base to an acidic solution of a sodium silicate solution, and reacting under an acid.

In the precipitation method of silica, a precipitated silicic acid slurry was made by growing or agglomerating secondary particles so as to have structural characteristics, where the secondary particles comprise primary particles generated by the neutralization of the aqueous solution of alkali metal silicate and the mineral acid, and the product was made by filtrating and drying, and/or continuously pulverizing, and / or classifying the slurry as indicated in Japanese Patent Gazette No. Syou 39-1207. That is, after static drying or spray drying, silica is pulverized by a suitable pulverizer according to the object, for example, a pin mill, in which the silica particles are pulverized by impact shearing and friction force between pins, or a jet pulverizer, in which the silica particles are pulverized by a high pressure jet stream and impacting each other, as indicated in "The newest ultrafine pulverizing process technique", edited by the Publication Division of the Soft Giken Company, and published by the New Technique Information Center, 1985, pages 8 to 10. As for silica by the general precipitation method, a BET specific surface area is within the range from 80 to 450 m$^2$/g in general, and about 600 m$^2$/g in special cases. In general, silica by the precipitation method has a comparatively large pore and high oil absorption. Silica by the precipitation method is mainly used as a filler for reinforcing rubber, an adsorption carrier for agrochemicals, a matting agent for coating material, a viscosity adjusting agent for various mediums, or the like.

On the other hand, silica can be made by the gel method, for example, by washing and drying the gel made by the acid reaction, and pulverizing, as indicated in US 2,466,842. Making silica by the gel method makes for strongly agglomerated secondary particles, which are smaller than silica made by the precipitation method in general, and can keep the structural characteristics even when the particles are sheared under a high load, so that silica by the gel method is effectively used for applications such as a coating for leather, plastics or the like. Further, since silica made by the gel method has lower bulkiness and higher fluidity than silica made by the precipitation method in general, it is also used as an adsorbent for pharmaceuticals, agrochemicals or animal medicines, or the fluidity improving agent of powder.

However, although silica made by the precipitation method is used effectively as a matting agent in, for example, a coating material field because it has high oil absorption, its effects may be decreased remarkably under use, when the silica particles are dispersed under high share stress, for example, in a sand mill or the like, considering the importance of the surface condition of the coated film. Further, when silica made by the precipitation method is used as the adsorbent for pharmaceuticals, agrochemicals or animal medicines, the adsorbing ability is often decreased by pulverizing with a stirrer, mixer or pulverizer in the production line, and thus the adsorbed chemicals may leak, and further, adsorb into the walls of the production apparatus, or close the line of the production apparatus. As a cause of these problems, it is considered that the pores of the amorphous silica particles are comparatively large and crushed easily.

On the other hand, silica made by the gel method maintains the structural characteristics even when the particles are sheared under a high load since having a comparatively small and strong pore structure, however, a strong acid range, in which the pH is 1 to 2, is necessary to mix and/or react the aqueous solution of the alkaline metal silicate and the mineral acid. Further, washing particles is generally difficult, and a long time, that is 36 to 48 hours, is necessary to wash the particles for completely removing salts, such as Glauber's salt or the like, which is by-produced in hardened and blocked hydrogel. Therefore, in the production equipment, a corrosion resistant material that can withstand a strong acidity atmosphere is required, and washing for a long time is needed, and thus plant-and-equipment investment and production costs are increased. Moreover, as for silica made by the gel method, the pores are small, and the oil absorbing rate is relatively low, and so when silica made by the gel method is especially used as the chemical absorbing (adsorbing, oil absorbing) agent, such as for pharmaceuticals, agrochemicals, animal medicines, bathing agents or the like, it takes a long time to absorb the chemical liquid into silica. In such a case, it is necessary to drop the liquid component slowly and quite carefully, and if such a condition is unfavorable, the powder containing the chemical liquid and the amorphous silica is strongly agglomerated to increase the viscosity, and thus production troubles occur, such as the production line clogging or the stirring mixer stopping. So the desired absorbed silica can not be made. Furthermore, since the particles itself are hard, the equipment is easily worn when the particles are mixed or pulverized with a raw material as, for example, as the adsorbent or matting agents for pharmaceuticals, agrochemicals, animal medicines, and thus an improvement was required.

It was an object of the present invention, therefore, to provide new silica capable to solve at least some of the aforementioned problems. In particular, to provide amorphous silica particles, in which the oil absorbency is not easily decreased and the oil absorbing rate is high, even when under a high shear stress, that is, a high load is applied to the amorphous silica particles. The intention is likewise to provide a process for preparing the silicas of the invention.

For solving the above mentioned problems, wholehearted investigations were carried out to make the amorphous silica particles, in which the oil absorbency is not easily decreased and the oil absorbing rate is high, even when under a high shear stress, that is, a high load is applied to the amorphous silica particles. In this method, the fabrication method of silica by the precipitation method was used since it is simple and economical from the viewpoint of the equipment and process. As a result, the present inventors found out that there is an important correlation between the oil absorbency and the pore structure of the amorphous silica particles, by using the benzene adsorption isotherm method, in which a mesopore having a pore radius of 15 nm or more can be measured comparatively accurately. That is, it was found out that the oil absorbency is hardly decreased and the high oil absorbing rate is kept even when a high load is applied to the amorphous silica particles, when the maximum value of $\Delta Vp/\Delta Rp$ (where $Vp$ is the pore volume [$mm^3$/g] and $Rp$ is the pore radius [nm]) is 20 $mm^3$/nm·$g^{-1}$ or more in the pore distribution curve obtained by the benzene adsorption isotherm, and the pore peak radius when the $\Delta Vp/\Delta Rp$ value is maximum is from 20 nm or more to 100 nm or less.

The present invention therefore provides amorphous silica and a process for their manufacture as defined in the claims and the detailed description.

The present invention particularly provides amorphous silica particles, wherein the maximum value of $\Delta Vp/\Delta Rp$ (where $Vp$ is the pore volume [$mm^3$/g] and $Rp$ is the pore radius [nm]) is 20 $mm^3$/nm·$g^{-1}$ or more in the pore distribution curve obtained by a benzene adsorption isotherm, and the pore peak radius when the $\Delta Vp/\Delta Rp$ value is maximum is from 20 nm or more to 100 nm or less.

The present invention further provides a process for preparing amorphous silica according to the invention, wherein least one alkali metal silicate and at least one mineral acid are neutralized.

The present invention also provides the use of amorphous silica according to the invention for example as matting agent, adsorbent (carrier) for pharmaceuticals or agrochemicals, extender, or filler of various rubbers or the like.

The present invention in addition provides matting agents and adsorbents for pharmaceuticals and agrochemicals comprising the amorphous silica paticles of the invention.

The amorphous silica particles of the invention exhibit a high oil absorption, that is hardly decreased even when high shear forces act on the silica particles. Since the amorphous silica particles of the invention have high oil absorbency, a high amount of vitamin E can be powdered with a little amount of the amorphous silica powder. In addition, the matting effect is higher than that of the commercially available silica particles. As consequence, these amorphous silica particles can be used as an adsorbent for pharmaceuticals or agrochemicals, an extender, or a filler of various rubbers or the like.

Although an alkaline silicate being a raw material of the amorphous silica particles of the present invention is not limited especially, the following can be used, that is, sodium silicate or potassium silicate, such as water glass standardized according to JIS as an industrial product, an alkali silicate made by reacting a readily reactive silica with a hydroxide solution of an alkaline metal, or the like, where the reactive silica is recovered from a clayey raw material, such as acidic clay or the like. When the above alkali silicate is used as an aqueous solution, the silica concentration of the aqueous solution is not limited especially but is generally 1 to 30 % by weight, preferably 2 to 20 % by weight, and more preferably 2.5 to 10 % by weight. when the concentration is less than 1 % by weight, the production efficiency becomes low and the economical disadvantageous increases. When the concentration is more than 30 % by weight, the viscosity of the reaction solution becomes high, the reaction loses uniformity, and thus the handling of silica slurry after the reaction becomes very difficult. Furthermore, the mol ratio of $SiO_2$:$M_2O$, where M is an alkaline metal, is 2:1 to 4:1 generally, and preferably 2.5:1 to 3.5:1. These mole ratios are called No. 2 diatom, No. 3 diatom, No. 4 diatom, or the like. In general, the No. 3 diatom is preferably used for its cost effectiveness.

As the mineral acid used for neutralization in making the amorphous silica particles, carbonated water, carbonated gas, acetic acid, Lewis acid, hydrochloric acid, sulfuric acid, nitric acid or the like can be used although it is not limited especially. Particularly, the sulfuric acid is preferably used from the viewpoint of equipment and economy. The concentration of the mineral acid aqueous solution is generally 5 to 75 % by weight, preferably 10 to 60 % by weight, and more preferably 10 to 45 % by weight.

The silica of the invention may be produced by a process, wherein an aqueous solution of alkali metal silicate is neutralized with an acid. As a method for the neutralization by contacting both raw materials, there are two methods, that is, the method where one of the raw materials is added to the aqueous solution of another raw material while stirring, and the method where both solutions of raw materials are contacted simultaneously under the fixed condition. The making examples are shown in the following.

As described above the amorphous silica of the invention can be produced preferably using a precipitation method. A gel method or a combination of gel- and precipitation-method may be suitable, too. In this cases, especialla when a combined method is used, it is necessary to control the growths and agglomerations of the amorphous silica particles, which are used as a nucleus generated in the first stage reaction, and the silica particles aged after this generation. That is, it is necessary to decide the fabrication conditions, considering the particle size or the pore size of the silica particles used as the nucleus, and the particle size and the pore size of the silica particles after aging.

In a first preferred embodiment the process of the invention comprises, a first step, wherein an alkaline silicate aqueous solution and a mineral acid aqueous solution are neutralized in a pH of 2 to 10 to directly make a silica slurry having 2 to 10 % silica concentration. Or a silica slurry is made by the neutralization of the alkaline silicate aqueous solution and the mineral acid aqueous solution having a 5 to 30 % silica concentration by weight by leaving for 30 minutes or more in general. The neutralization temperature is preferably 50 ˚C or less in general from the viewpoint of silica having a uniform texture, although it is not limited especially. Furthermore; the neutralization may be carried out while applying the shearing force by a wet type pulverizer or the like, according to the necessity. In this case, the neutralization temperature is about 70˚C although it is changed with time.

After the obtained silica is washed, a heating treatment may be carried out for a moisture adjustment and a pore adjustment according to necessity. Although the conditions are not especially limited, the heating treatment has to be carried out so as to become the pore peak radius being within the range from 20 to 100 nm finally. The amorphous silica particles made by this method have generally a pore peak radius of 10 nm or less, and is preferably heat-treated at a higher temperature and longer time than the conventional method, and within the high pH range of 5 to 9 according to necessity. For example, the temperature of the heating treatment is generally 40 to 200 ˚C , preferably 70 to 190 ˚C , and more preferably 100 to 170 ˚C. For example, the heating treatment can be carried out, for example, in an autoclave, and the time for the heating treatment may be adjusted according to the pore peak radius. The time is generally 5 minutes to 30 hours, preferably 30 minutes to 20 hours, and more preferably 1 hour to 15 hours.

Then, the silica slurry may be wet-pulverized so as to have an average particle size of 500 $\mu$m or less, preferably 2 to 200 $\mu$m, and more preferably 3 to 100 $\mu$m, according to necessity. The silica slurry may be coarsely pulverized before the heating treatment or during the heating treatment according to the case, but the filtration efficiency is insufficient, and the silica slurry may be agglomerated again when it is compressed at the filtration, so that the silica slurry has to be repulverized after the filtration in this case.

As for the wet type pulverization, a commonly known method itself can be applied. For example, a bead mill, such as a dyno-mill made by WAB Company, a high shear mixer made by Silverson Company, a homo mixer or a line mill made by Tokushukika Company or the like can be suitably used. If the high speed shearing force is possible, other wet type pulverizers can also be used. The temperature at the time of the wet type pulverization is not particularly limited, but when the pulverization is carried out during the reaction or the heating treatment, it can be carried out under the same temperature. However, when the pulverization is carried out after ending the pore size adjustment, the temperature of the slurry must be less than 50 ˚C to decrease the agglomeration between particles.

Then, the predetermined amorphous silica can be obtained by filtrating the silica slurry and drying. As a drying method, the commonly known method such as air-drying or spray-drying can be used. In general, when a highly oil absorbing silica is desired, a spray dryer or a spin flush dryer capable of drying for a short time is preferably used. In the case of the spray dryer, two methods are used generally for finely atomizing the slurry, that is, one is using a spray disc (atomizer), the other is using a two-fluid nozzle, but it is not particularly used in the present invention. In addition, when the slurry is dried by the spray dryer, an almost spherical solid particle can be made. The temperature of the hot air of the spray dryer is 80 to 600 ˚C, preferably 100 to 500 ˚C, and more preferably 120 to 450 ˚C. In order to improve the oil absorption, it is more advantageous that the temperature of the hot air is high, however, when the temperature is 600 ˚C or more, the production cost of the dryer becomes high in order to have heat resistance and a special facility design. On the other hand, when the temperature is 100 ˚C or less, the production efficiency is insufficient. In particular, it can be optimized with the relationship between the performance of the spray dryer and the spraying speed, but the above-mentioned temperature range is preferable in general. Furthermore, the moisture can be easily removed from the surface of the particles in an aqueous phase and shrinkage of the amorphous silica particles during the drying process can be effectively controlled by adding a cationic surfactant, such as alkyldimethyl benzyl - ammonium chloride or the like, to the slurry before drying according to necessity, and thus the oil absorption can be increased.

In a preferred embodiment 2 the process of the invention comprises a step of neutralizing at least one alkaline silicate aqueous solution with at least one acid aqueous solution in a pH range of 5 to 10 to make the silica slurry having 2 to 10 % by weight silica concentration. In this case, types, concentrations and the neutralization method of the alkaline silicate aqueous solution and the mineral acid aqueous solution are the same as above-mentioned method. The neutralization temperature is preferably 30 ˚C or more, more preferably 50 ˚C or more, and furthermore preferably 70 ˚C or more, although it is not limited especially. When the temperature is less than 30 ˚C, the reaction rate is slow, and thus it is not efficient. Further, the neutralization can be carried out while applying the searing force by a wet type pulverizer mentioned above or the like, according to necessity. Then, the generated silica slurry can be aged according to its physical properties. As for the general aging conditions, the pH is 6 to 12, the temperature is 50 to 130 ˚C, and the reaction time is 3 to 180 minutes. Preferably, pH is 7 to 11.5, the temperature is 60 to 110 ˚C and the reaction time is 3 to 165 minutes. More preferably, pH is 8 to 11, the temperature is 65 to 100 ˚C and the reaction time is 5 to 150 minutes. Especially preferably, the pH is 8 to 11, the temperature is 70 to 100 ˚C and the reaction time is 5 to 140 minutes. Further, the silica slurry can be aged while applying the shearing force by a wet type pulverizer mentioned above or the like, according to the necessity.

Further, as the second stage reaction, the mineral acid can be added simultaneously to the slurry made by the first stage reaction while adding the sodium silicate aqueous solution. In this case, although the concentration of the mineral acid added for the second stage reaction is within the same concentration range as that of the first stage reaction, it is preferable that the concentration of the sodium silicate aqueous solution is within the same range as that of the first stage reaction or lower. Further, the pH at the second stage reaction is preferably fixed, and is generally 4 to 10, preferably 6 to 10, and more preferably 7 to 9.5. Then, pH of the obtained silica slurry is adjusted to 4 or less, preferably to 3 or less, and then, the second stage reaction is stopped. According to necessity, the slurry is diluted with water, and the coarse particles are separated by a rotary pump and a hydrocyclone if necessary, and then the slurry is filtrated and washed. The filtration and washing can be carried out by using a commonly known instrument, such as a filter press, a rotary filter or the like.

The filter cake obtained in this way is pulverized to have the suitable size, and slurried again by carrying out the air-drying or stirring while adding water. Then, the slurry solution can be dried by the spray dryer, the nozzle dryer or the like. The specified particle size distribution can be adjusted by the dryer. This distribution can be adjusted as per the type of dryer and the selection of an applied spraying pressure. In order to make the especially highly oil absorbing silica, the drying is preferably carried out by the spray dryer. When the spray dryer is used, the drying can be carried out under the same conditions as the above-mentioned conditions.

Moreover, as for the pH of the obtained silica of embodiment 1 or 2, the suitable pH is changed according to the application. More particularly, when the silica is used as the adsorbent of pharmaceuticals or agrochemicals, pH influences the stability of a pharmaceutical active ingredient, such as vitamin E or the like, and an agrochemical active ingredient, such as an organophosphorus agent or the like, and is very important. The pH of the amorphous silica particles when these are used as the adsorbent of pharmaceuticals or agrochemicals is generally 3 to 10, preferably 4 to 9, and more preferably 5 to 8. However, the pharmaceuticals or agrochemicals adsorbed in the silica can be stabilized by applying the adjusted silica, according to the case, that is, applying the silica adjusted to acid in the case of the compound being stable in acid, and applying the silica adjusted to alkaline in the case of the compound being stable in alkaline. As the method for adjusting the pH, there are two methods, that is, a method adjusting the pH of the silica slurry before drying, and a method adjusting the pH by adding ammonia gas or the like after drying.

The amorphous silica of the invention are characterized in that the maximum value of $\Delta Vp/\Delta Rp$ (where Vp is the pore volume [$mm^3/g$] and Rp is the pore radius [nm]) is 20 $mm^3/nm\cdot g$ or more in the pore distribution curve obtained by a benzene adsorption isotherm, and the pore peak radius when the $\Delta Vp/\Delta Rp$ value is maximum is from 20 nm or more to 100 nm or less, preferably from 25 nm or more to 95 nm or less, and more preferably from 30 nm or more to 90 nm or less. When the pore peak radius is 20 nm or less, the silica particles are agglomerated strongly to have a small pore and decreases the oil absorbing rate. Further, since the silica particles becomes to hard, the wear of the equipment may become a problem. On the other hand, the pore is 100 nm or more, the particles have high structural characteristics, the agglomeration between particles is low, and thus the particles itself may be easily collapsed.

That is, the strength of the particles can be adjusted by synthesizing the silica having a pore structure in which the pore radius is from 20 nm or more to 100 nm or less, and the adsorbing and absorbing rate by a capillary phenomenon can be improved. Further, the maximum value of $\Delta Vp/\Delta Rp$ (where Vp is the pore volume [$mm^3/g$] and Rp is the pore radius [nm]) is 20 $mm^3/nm\cdot g$ or more, preferably 25 $mm^3/nm\cdot g$ or more, and more preferably 30 $mm^3/nm\cdot g$ or more. When the maximum value of $\Delta Vp/\Delta Rp$ (where Vp is the pore volume [$mm^3/g$] and Rp is the pore radius [nm]) is 20 $mm^3/nm\cdot g$ or less, there is almost no structural difference between the silica of the present invention and the silica having an open structure and not having the maximum peak, and the oil absorbency under the high shear stress may be remarkably decreased.

The silicas according to the present invention can exhibit an oil absorption, measured by JISK 6217-4 (a Brabender method), in which the oil absorption of DBP (dibutylphthalate) is used as an index, of more than 260 ml per 100g of the amorphous silica particles (260 ml/ 100g), preferably 280 ml (280 ml/ 100g) or more, more preferably 300 ml (300 ml/ 100g) or more, furthermore preferably 320 ml (320 ml/ 100g) or more, and especially preferably 340 ml (340 ml/ 100g) or more.

In the JIS test, although the DBP dropping rate is fixed at 4 ml/minute, the dropping rate is generally correlated with the oil absorption. In general, when the DBP dropping rate becomes high, the stirring time until the end of the measuring by the Brabender method becomes short, and the total quantity of the load to the amorphous silica particles is decreased, and thus the amorphous silica particles is not easily collapsed. Therefore, since the pore structure can be easily maintained, the oil absorption is increased in general as compared with the silica particles treated under conventional conditions. That is, it can be said that the particles, where the oil absorption is increased remarkably when the DBP dropping rate is changed, is the structurally weak particles.

On the other hand, in the Brabender method, the change of the viscosity of the oil-absorbed silica is measured with the change of the torque. Therefore, when the DBP dropping rate becomes high, the DBP amount dropped during the time lag, that is time necessary for silica to absorb DBP and increase viscosity, is increased, and thus the oil absorption is apparently increased. In other words, if the oil absorption is remarkably increased when the DBP dropping rate is increased, the particles have a low oil absorbing rate. That is, as the factors influencing the oil absorption when the DBP dropping rate is changed, there are two factors, that is, the structural strength and the DBP oil absorbing rate, and the oil absorption of the silica particles having the strong structure and high DBP oil absorbing rate is not easily influenced with the DBP dropping rate. However, in general, although the amorphous silica particles having a large pore radius has a weak structure, the oil absorbing rate of these particles is high, and although the amorphous silica particles having the small pore radius has a strong structure, the oil absorbing rate of these particles is low. Then, as for the structural factor which is strong or weak and the factor of the oil absorbing rate; the influence to the oil absorption is considered when the DBP dropping rate is changed from low to middle to high. The structural factor is strongly influenced with the stirring time and the factor of the oil absorbing rate is strongly influenced with the dropping rate. That is, as for the influence to the oil absorption, it can be considered that the influence of the structural factor is larger than that of the factor of the oil absorbing rate when the dropping rate is in a range from low to middle, and the influence of the factor of the oil absorption is larger than that of the structural factor when the dropping rate is in a range from middle to high.

Then, an index of the oil absorption is obtained from the following two formulas when the DBP dropping rate, that is, the load applied to the amorphous silica particles, is changed.

$$OI\,1\left[\frac{\min}{100g}\right] = \frac{OA\,[ml/100g]\,at\,DR=7\,[ml/\min]-OA\,[ml/100g]\,at\,DR=2\,[ml/\min]}{5\,[ml/\min]} \quad \text{Formula (I)}$$

DR = dropping rate; OA = Oil Absorption

When the OI1 value of the formula (I) is low, the amorphous silica particles have a hard and strong structure and high oil absorbing rate.

$$OI\,2 = \frac{OA\,[ml/100g]\,at\,DR=7\,[ml/\min]-OA\,[ml/100g]\,at\,DR=4\,[ml/\min]}{OA\,[ml/100g]\,at\,DR=4\,[ml/\min]-OA\,[ml/100g]\,at\,DR=2\,[ml/\min]} \quad \text{Formula (II)}$$

The formula (I) is the formula, in which the influence of the structural strength and the influence of the oil absorbing rate are doubled. In both of the denominator and numerator in the formula (I), although the influence of the structural strength and the influence of the oil absorbing rate are doubled, the influence of the structural factor is strong in the denominator, and the influence of the oil absorption easily occurs in the numerator. Therefore, the influence of the oil absorbing rate can be evaluated to decrease the influence of the structural factor by the formula (II). That is, when the value of formula (II) is less than 1, it can be said that the amorphous silica particles have a high oil absorbing rate after removing the structural factor of the particles.

The amorphous silica particles of the present invention may therefore be characterized by an oil absorption index 1 (OI1) according to formula (I), which is the total index of the change of the oil absorption to the DBP dropping rate, that is generally 9.5 [min/100g] or less, preferably 9 [[min/100g] or less, more preferably 8.5 [min/100g] or less, furthermore preferably 8 [min/100g] or less, and especially preferably 6 [min/100g] or less.

On the other hand they may be characterized by oil absorption index 2 (OI2) acording to formula (II), which is the index value of the oil absorbing rate, that is generally 1.2 or less, preferably 1.1 or less, more preferably 1.0 or less, and furthermore preferably 0.8 or less. respectively 0.7 or less

If oil absorption indexes 1 and 2 are simultaneously within the above ranges, it is said that the amorphous silica particles have a strong particle structure and a high oil absorbing rate.

The amorphous silica of the invention may exhibit a BET specific surface area in the range of 50 to 800 $m^2/g$, preferably 100 to 700 $m^2/g$, and more preferably 140 to 400 $m^2/g$. The BET specific area is one of the basic properties of amorphous silicas, and influences the oil absorption, the transparency of the particles and the handling of the amorphous silica particles. The inventors found out, that when the BET specific surface area is less than 50 $m^2/g$, there are little amounts of the large size pore, and the pore is collapsed easily by the load to the amorphous silica particles, and thus the oil absorption performance under the shear stress is decreased. Further, the matting effect may be decreased since the transparency of the amorphous silica particles is decreased. On the other hand, when the BET specific surface area is more than 800 $m^2/g$, the pore size is very small, and thus the oil absorbency is decreased although the transparency is increased.

The amorphous silica obtained according to the process of the invention can be merchandised as it is, but the particle size of the silica can be adjusted according to the application. The particle size can be adjusted by carrying out a dry classification after pulverizing. As the pulverizer, it is not especially limited, and all commonly known pulverizers can be used, for example, an air current impact type pulverizer, such as Jet - O- Mizer or the like, a hammer mill, such as an atomizer or the like, a pin mill, such as a centrifugal classifier or the like, can be used. As a classifier, although it is not especially limited, a dry classifier, such as a microplex, a turbo classifier or the like, is suitable when a precise classification is required. On the other hand, the silica slurry after washing can be dried after classifying by a wet classifier, such as a precipitation classifier, a hydraulic classifier, a mechanical classifier, a centrifugal classifier or the like. More particularly, when the silica is used as the filler for an ink jet recording paper, the matting agent, an antiblocking agent or the like, the adjustment of the particle size is important. Therefore, the silica of the invention may exhibit a median size which is based on volume and the average particle size of the amorphous silica particle of the present invention, within the range from 0.5 to 100 $\mu$m, preferably from 0.75 to 50 $\mu$m, and more preferably from 1 to 20 $\mu$m. If the median size is within the above range, a matting function and an antiblocking function can be realized with few blending amounts.

The silica of the present invention may in addition be characterized by their bulk density. The bulk density is a very important physical property for handling the amorphous silica particles. The bulk density of the amorphous silica particles of the present invention may be 20 to 200 g/l, preferably 30 to 150 g/l, and more preferably 40 to 125 g/l. When the bulk density is less than 20 g/l, the handling may be difficult since the bulk becomes very high, and when the bulk density is more than 200 g/l, the usage amounts are increased in various applications, and the oil absorption may be decreased.

The forementioned physical-chemical properties may be combined independently. Particular preferred combinations are described in the following paragraphs.

As for the physical properties of the amorphous silica particles of the present invention, it is especially preferable that the maximum value of $\Delta Vp/\Delta Rp$ (where Vp is the pore volume and Rp is pore radius) is 20 $mm^3/nm\cdot g$ or more in the pore distribution curve obtained by the benzene adsorption isotherm, the pore peak radius when the $\Delta Vp/\Delta Rp$ value is maximum is from 20 nm or more to 100 nm or less, the oil absorption measured by JISK 6217-4 (the carbon black for rubber - basic characteristics) is 280 ml/100g or more, the index value being the change of the oil absorption to the DBP dropping rate (the OI1) is 9.5 [min/100g] or less, and the index value being the oil absorbing rate (the 012) is 1.2 or less. More preferably, the maximum value of $\Delta Vp/\Delta Rp$ is 25 $mm^3/nm\cdot g$ or more, the pore peak radius when the $\Delta Vp/\Delta Rp$ value is maximum is from 25 nm or more to 95 nm or less, the oil absorption measured by JISK 6217-4 (the carbon black for rubber - basic characteristics) is 300 ml/ 100g or more, the OI1 is 9 [min/ 100g] or less, and the 012 is 1.0 or less.

As for the physical properties of the amorphous silica particles of the present invention, more particularly, it is preferable that the maximum value of $\Delta Vp/\Delta Rp$ is 20 $mm^3/nm\cdot g$ or more, the oil absorption measured by JISK 6217-4 (the carbon black for rubber - basic characteristics) is 280 ml/100g or more, the OI 1 is 9.5 [min/100g] or less and the OI 2 is 1.2 or less, the BET specific surface area is 50 to 800 $m^2/g$, the average particle size is 0.5 to 100 $\mu$m, and the bulk density is 20 to 200 g/l. More preferably, the maximum value of $\Delta Vp/\Delta Rp$ is 25 $mm^3/nm\cdot g$ or more, the pore peak radius when the $\Delta Vp/\Delta Rp$ value is maximum is from 25 nm or more to 95 nm or less, the oil absorption measured by JISK 6217-4 (the carbon black for rubber - basic characteristics) is 300 ml/ 100g or more, the OI1 is 9.0 [min/ 100g] or less and the OI value 2 is 1.1 or less, the BET specific surface area is 100 to 700 $m^2/g$, the average particle size is 0.75 to 50 $\mu$m, and the bulk density is 30 to 150 g/1. Even more preferably, the maximum value of $\Delta Vp/\Delta Rp$ value is 30 $mm^3/nm\cdot g$ or more, the pore peak radius when the $\Delta Vp/\Delta Rp$ value is maximum is from 30 nm or more to 90 nm or less, the oil absorption measured by JISK 6217-4 (the carbon black for rubber - basic characteristics) is 320 ml/ 100g or more, the OI1 is 8.5 [min/ 100g] or less and the OI2 value is 1.0 or less, the BET specific surface area is 140 to 450 $m^2/g$, the average particle size is 1 to 20 $\mu$m, and the bulk density is 40 to 125 g/l.

The amorphous silica particles of the invention, in which the oil absorption is hardly decreased even when applying a high load, were invented. As for the amorphous silica particles of the present invention, since they have high absorbency, high amounts of the liquid chemicals, such as agrochemicals, feed, cosmetics, perfume, detergent, liquid vitamin or the like, can be powdered with small amounts of the amorphous silica powder. In addition, the matting effect is higher than that of the commercially available particles. The amorphous silica particles of the present invention can be used as matting agent, for coating materials or the like, extender of agrochemicals, and reinforcing agent of various or the like.

The amorphous silica particles of the present invention are particularly used as carrier respectively adsorbent, i. e. for blocking blow film, improving the fluidity or the storage stability of the powder, solidification of a liquid component. In addition the silica of the invention show excellent performances when used as matting agent or as reinforcing agent.

The silica of the invention are in particular useful in the field of pharmaceuticals, agrochemicals and bathing agents. The amorphous silica particles are used as the adsorbent, the powdering agent, the extender, the caking preventing agent, the fluidity improving agent, or a pulverizing auxiliary of the liquid component of vitamin A, vitamin E, a pharmaceutically active ingredient, a pyrethroid, an organophosphorus agent, a herbal medicine extracting component or the like. For example, when the vitamin E is powdered, vitamin E of preferably 2.2 times or more by a weight ratio, more preferably 2.3 times or more, and even more preferably 2.4 times or more can be adsorbed in 100 g of the amorphous silica particles of the present invention. Further, the silica particles can be used as a stabilizing agent by adjusting the pH of the silica according to the stability of an active ingredient. In the agrochemicals field, in addition to the usage method, the silica particles can be used as the precipitation preventing agent in each floatable agent, and a validity-strengthening agent according to the case.

When the amorphous silica particles of the present invention are used as a carrier for agrochemicals, they can be applied to all commonly known dosage forms by mixing with an agrochemical technical product, but is not especially limited. In addition, in the field where a conventional precipitating silica is used, the amorphous silica particles can be used satisfactorily. For example, the following formulations can be used, that is, a fine powder-like formulation, such as powder granules, wettable granules or the like, a powder-like formulation, such as granules, powdery granules, granular wettable granules or the like, a solid formulation such as a tablet or the like, a uniform solution-like formulation, such as a solution, an oil solution, an emulsion, a micro emulsion or the like, or an emulsification or suspension-like formulation, such as suspension in water, suspension in oil, emulsion in water, emulsion in oil, microcapsule or the like. Each formulation can be made by the commonly known composite and production method.

For example, in the case of a solid formulation, when the agrochemical technical product is solid and the other auxiliary component is solid, the silica particles can be used as, for example, the pulverization auxiliary, the fluidity improving agent, a powder explosion decreasing agent, a caking preventing agent or the like. When the agrochemical technical product is liquid or semi-solid, or contains a solvent or the like in the formulation, the silica particles can be used as, for example, the adsorbent of the agrochemical technical product, the solvent or the like. Moreover, in the case of the liquid formulation, the silica particles can be used as, for example, the viscosity modifier for preventing the precipitation, or the fluidity improving agent of the solid component mixed in the liquid. Furthermore, in the case of mixing the solid component after pulverization, the silica particles can be used as, for example, the pulverization auxiliary, the fluidity improving agent, the powder explosion decreasing agent or the like.

A especially preferred use of the silica of the invention is as matting agent. In this field of use, the amorphous silica particles itself can be blended with a commonly known coating material to become a matte coating composition. As the coating material, the commonly known and commonly used coating materials can be used, that is, for example, an oil coating material, a nitrocellulose coating material, alkyd resin coating material, an amino alkyd coating material, a vinyl resin coating material, an acrylate resin coating material, an epoxy resin coating material, a polyester resin coating material, a chlorinated rubber-base coating material or the like. Further, in addition to these materials, the coating material containing one or more kinds of the following resins can be used, that is, a rosin, an estergum, a pentaresin, a coumarone indene resin, a phenol-based resin, a modified phenol-based resin, a maleic-based resin, an alkyd-based resin, an amino-based resin, a vinyl-based resin, a petroleum resin, epoxy-based resin, a polyester-based resin, a styrene-based resin, an alkyl-based resin, a silicone-based resin, a rubber-based resin, a chloride-based resin, an urethane-based resin, a polyamide-based resin, polyimide-based resin, a fluorine-based resin, a nature or synthetic Japanese lacquer or the like.

Further, as for the coating material under use, although a solution-type coating material, a water-based coating material, an ultraviolet curable coating material, a powder coating material or the like can be used arbitrarily, the present invention is especially suitable for a solution type coating material and a water-based coating material.

As an organic solvent of the solution type coating material, one or more types of the following solvents can be used, that is, an aromatic hydrocarbon-based solvent, such as toluene, xylene or the like, an alicyclic hydrocarbon-based solvent, such as cyclohexane or the like, a ketone-based solvent, such as acetone, methylethyl ketone, methylisobutyl ketone, cyclohexanone or the like, an alcohol-based solvent, such as ethanol, propanol, butanol, diacetone alcohol or the like, an ether-based solvent, such as tetrahydrofuran, dioxane or the like, a Cellosolve-based solvent, such as ethyl Cellosolve, butyl Cellosolve or the like; an ester-based solvent, such as ethyl acetate, butyl acetate or the like, an aprotic polar solvent, such as dimethylformamide, dimethylacetamide, dimethylsulfoxide or the like. A resin content concentration in a raw material solution is generally within the range from 5 to 70 % by weight, suitably from 10 to 60 % by weight.

Further, as a water-based coating material, a self-emulsifying or a surfactant-emulsifying coating material is used, other than the water-based solution type coating material. As a resin of the water-based coating material, the following resins being water-solubilized or self-emulsified to the water-based solvent can be used, that is, an alkyd resin, a polyester resin, an acrylic resin, an epoxy resin, or a mixture of two or more kinds of these resins. In the self-emulsifying resin, the self-emulsifying property is given by neutralizing a carboxyl group with ammonias or amines or quaternizing the contained amines. Further, various latex resins are also used. The resin content concentration is generally within a range from 10 to 70 % by weight, especially suitable from 20 to 60 % by weight.

As the ultraviolet (UV) curable coating material, the following resins can be used, that is, a high solid resin, for example, a UV curable type-acrylic resin, -epoxy resin, -vinyl urethane resin, -acrylic urethane resin, and - polyester resin. These resins are used independently or by mixing more than two kinds.

As the powder coating material, the following can be used, that is, a thermoplastic resin, such as polyamide, polyester, an acrylic resin, an olefine resin, a cellulosic derivative, polyether, a vinyl chloride resin or the like, an epoxy resin, an epoxy/novolak resin, an isocyanate or epoxy curable polyester resin or the like.

As for the amorphous silica particles used for the present invention, the surface of the silica particles can be coated or surface-treated with an inorganic oxide, such as titanium oxide, silicon oxide, zirconium oxide, zinc oxide, barium oxide, magnesium oxide, or calcium oxide, or a coupling agent such as a silane-based, titanium-based or zirconium-based coupling agent.

Moreover, as for the amorphous silica of the present invention, the coating of waxes can be carried out with the request material from a metallic soap, a resin acid soap or various resins. More particularly, the wax treatment by an olefin-based resin wax, such as a polyethylene wax, an oxidation polyethylene wax or an acid-modified polyethylene wax, an animal and vegetable wax, a mineral-based wax or the like is effective for increasing the matting effect or improving scratch resistance. The coating treatment can be carried out easily by adding an aqueous emulsion of the wax to the cake of the washed amorphous silica and mixing. The weight ratio of the surface-treated wax to the amorphous silica is 1 to 20 %, where the amorphous silica is 100 %, preferably 3 to 15 %.

In the present invention, the amorphous silica particles can be not only independently used as matting agent, but also for blending the coating material with other filler or pigment. As the inorganic component blended with the coating material, the following can be used, that is, alumina, attapulgite, kaolin, carbon black, graphite, fine powdered silicic acid, calcium silicate, diatomaceous earth, magnesium oxide, magnesium hydroxide, aluminum hydroxide, slate powder, sericite, flint, calcium carbonate, talc, feldspar powder, molybdenum disulfide, barite, vermiculite, whiteing, mica, pyrophyllite clay, gypsum, silicon carbide, zircon, glass bead, shirasu balloon, asbestos, glass fiber, carbon fiber, rock wool, slag wool, boron whisker, stainless steel fiber, titanium white, zinc white, red oxide, iron black, yellow iron oxide, zeolite, hydrotalcite, lithium, aluminum, carbonate, titan yellow, chrome oxide green, ultramarine blue, Prussian blue, or the like.

A further especially preferred use of the silica of the invention is for blending a thermoplastic resin, a thermosetting resin or various rubbers, and especially, as an antiblocking agent. As the thermoplastic resin where the amorphous silica is blended as the antiblocking agent, an olefin-based resin is suitable, and especially, the following resins can be used, that is, polyethylene, isotactic polypropylene or syndiotacic polypropylenic, which have low, middle or high density, a polypropylene-based polymer being a copolymer of these ethylene and a-olefin, linear low density polyethylene, an ethylene-propylene copolymer, polybutene-1, ethylene-butene-1 copolymer, a propylene-butene-1 copolymer, an ethylene-propylene-butene-1 copolymer, an ethylene- vinyl acetate copolymer, an ion cross-linking olefin copolymer (ionomer), ethylene-acrylic ester coplymer, or the like. These resins can be used independently or in a blended-state by mixing two or more. The amorphous silica particles of the present invention are useful as the antiblocking agent of the olefin-based resin film made by using a metallocene catalyst, and can solve the coloration tendency of the conventional antiblocking agent.

Of course, the antiblocking agent of the present invention can be blended with other commonly known resin films. For example, the agent can be blended with a polyamide, such as nylon 6, nylon 6-6, nylon 6-10, nylon 11, nylon 12 or the like, thermoplastic polyester, such as polyethylene terephthalate, polybutylene terephthalate or the like, polycarbonate, polysulfone, a vinyl chloride resin, a vinylidene chloride resin, a fluoridation vinyl resin, or the like.

When the application is the antiblocking agent, the blending ratio of the silica particles to thermoplastic resin, where the thermoplastic resin is 100%, is 0.005 to 10 % by weight, preferably 0.05 to 3.0 % by weight, and more preferably 0.1 to 1.0 % by weight.

The amorphous silica particles of the present invention can be blended with the thermoplastic resin, various rubbers or thermosetting resin, as the filler.

As an elastomer polymer for rubber, for example, the following can be used, that is, nitrile-butadiene rubber (NBR), styrene butadiene rubber (SBR), chloroprene rubber (CR), polybutadiene (BR), polyisoprene (IIB), butyl rubber, natural rubber, ethylene propylene rubber (EPR), ethylene-propylene-diene rubber (EPDM), polyurethane, silicone rubber, acrylic rubber or the like, and further, the thermoplastic elestomer, such as a styrene-butadiene-styrene block copolymer, a styrene-isoprene-styrene block copolymer, a hydrogenation styrene-butadiene-styrene block copolymer, a hydrogenated styrene-isoprene-styrene block copolymer or the like.

As the thermosetting resin, the following resins can be used, that is, a phenol formaldehyde resin, a furan-formaldehyde resin, a xylene-formaldehyde resin, a ketone-formaldehyde resin, a urea-formaldehyde resin, a melamine-formaldehyde resin, an alkyd resin, a unsaturated polyester resin, an epoxy resin, a bismaleimide resin, a triallyl cyanurate resin, a thermosetting acrylic resin, a silicone resin, or a combination of two or more of these resins.

When the amorphous silica particles are used as filler, the silica particles can be blended with the thermosetting resin or elastomer within the range 0.5 to 20 % by weight, and preferably 2 to 10 % by weight, where the thermosetting resin or elastomer is 100%.

Beside the forementioned preferred applications, the silicas of the invention may be used as, a defoaming effect increasing agent for a defoaming material, a fluidity improving agent or a caking preventing agent of a powder fire extinguishing agent, a storage stability improving agent of the fluidity improving agent or caking prevention of various powders or the like, a filler of printing ink, a blur prevention agent of a newspaper ink, a purification adsorbent, a filter auxiliary agent for adsorbing proteins such as beer or the like, the powdering of the liquid component in feed, a milk extender, a fat conk, a milk powder, urea for drinks, a caking preventing agent for a caking substance such as a natural mixture or the like, a adsorbent of oil or fat of a feed for fish, a sintering prevention agent, a blocking prevention agent for a plastic industry or a blow film, such as polyethylene, polypropylene, PVC (polyvinyl chloride), HTV silicone rubber, a melamine resin, a phenol resin, a phenol- melamine resin or the like, a plate out preventing agent, a filler for polychloroprene rubber, thermoplastic rubber, silicone rubber or above-mentioned resins, an improving agent of mechanical characteristics of these flooring materials, an improving agent of measurement characteristics or caking preventing agent of these molded compounds, an adhesive auxiliary, a wear resistant improving agent, an improving agent of heat resistance/dimensional stability of TR crepe sole, a caking preventing agent of a foamed polystyrene granule preliminarily molded material, and a nucleating agent of a pattern constituting of a secondary molded film of styrene foam. Moreover, in a lacquer, varnish paint and mixture of these paints, the amorphous silica particles of the present invention are used as a partial replacement of titanium oxide or a white pigment in emulsion paint or ornament paint, a matting agent of a coating material, ink or the like, a precipitation preventing agent, a viscosity modifier, and a caking preventing agent.

Furthermore, in the field of the papermaking industry, the amorphous silica particles are useful as the partial replacement of titanium dioxide, the improving agent of a contrast of a blue printing paper, a coat agent for paper, and especially, a filler for the ink jet recording paper and a strike-through preventing agent for papermaking.

Moreover, the silica particles are used as powdering, the fluidity improving agent and the caking preventing agent of the surfactant, the filler of a battery separator, the auxiliary of adhesives, a thickening agent and auxiliary in toothpaste, a base material for adjusting a mol ratio of sodium silicate, powdering of chemicals for rubber, a powdery fluidity improving agent, a caking preventing agent or a heat-insulating material of refractories, a humidity modifier as itself or a coating agent to a wall, or a jet-flowability improving agent, caking preventing agent and tactual sense improving agent in food, or the like.

Furthermore, the amorphous silica particles of the present invention can be used as a chromatography carrier, a cosmetic base, a coating material for electronic parts, a moisture absorbent for electronic parts, and other applications of the amorphous silica particles.

**Examples**

Hereinafter, examples are indicated, but the present invention is not limited to these examples.

Example 1

A silica slurry having 10 % concentration was made by dropping 20 % sulfuric acid to 9000 1 of a 7.3 % sodium silicate solution at the rate of 43 1/minute and 95 ˚C for 30 minutes, adjusting pH to 4 immediately after the dropping, and filtrating washing. The obtained silica slurry was sprayed and dried using an atomizer type spray drier made by Ohkawara Kakohki Co. Ltd., and recovering the particles by cyclone. The recovered particles were pulverized using a mill (Type: Hammer Type Mill; Provider Fuji Sangyou (Japan)) and classified by a turbo classifier (Type: Air Classifier; Provider: Nisshin Engineering Inc.).

Example 2

A silica slurry was made by dropping 20 % sulfuric acid to 90001 of a 3.8 % sodium silicate solution while applying the shearing force at the rate of 21.5 1/minute and 95 ˚C for 30 minutes, aging it for 90 minutes, simultaneously adding a 9.8 % sodium silicate solution at the rate of 38.3 1/minute and 20 % sulfuric acid at the rate of 8.31 1/minute to the slurry for 75 minutes, keeping it at 95 ˚C for 30 minutes, and immediately adjusting the pH to 4. The obtained silica slurry was filtrated and washed to be adjusted to about 10 % slurry, and sprayed and dried by an atomizer type spray dryer made by Ohkawara Kakohki Co. Ltd., to be recovered by cyclone. The recovered particles were pulverized using a mill (Type: Hammer Type Mill; Provider Fuji Sangyou (Japan)) and classified using a turbo classifier classifier (Type: Air Classifier; Provider: Nisshin Engineering Inc.).

Comparison Examples 1 - 3

As for Comparison examples, commercially available amorphous silica particles were used (Comparison example 1 : Sylysia 350 [made by Fuji Silysia Chemical Ltd.], Comparison example 2 : Mizukaseal P-526 [made by Mizusawa Industrial Chemicals Ltd.], Comparison example 3 : SIPERNAT 50S [made by Degussa Co. Ltd.]).
Next, the measuring methods of each physical property were shown.

Test example 1

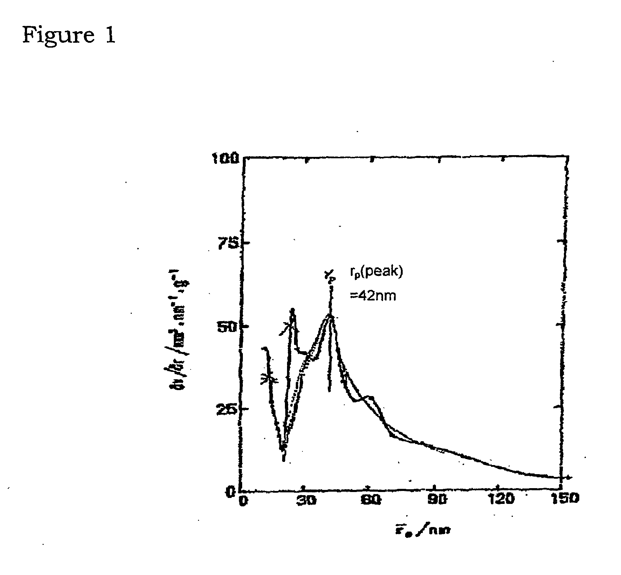

Measuring method of a benzene adsorption isotherm
For determining the pore structure of the amorphous silica particles of the invention, the pore peak radius was measured by the benzene adsorption method, in which mesopores having the pore radius of 20 nm or more can be measured.
In the measurement of the benzene adsorption isotherm, an automatic adsorption device, which was described in Figure 1 in Pharmatec Japan Vol. 12 No. 2 85-94 (1996), was used. Further, in the measurement, the sample, which was transferred to a sample tube, was carried out by the vacuum degassing treatment ($1\times10^{-2}$ Torr) at 25 °C for 8 hours, as the pretreatment of the sample.
The pretreated sample tube was set to the automatic adsorption device, and after measuring a dead volume, the adsorption isotherm was measured under the following conditions.

- Temperature conditions : Measuring temperature : 10°C, Thermostatic bath temperature : 40°C
- Adsorption equilibrium time : 20 minuets
- Adsorptive substance : Benzene (a saturated steam pressure at 10°C : 45.5 Torr)
- Analyzing the pore distribution curve

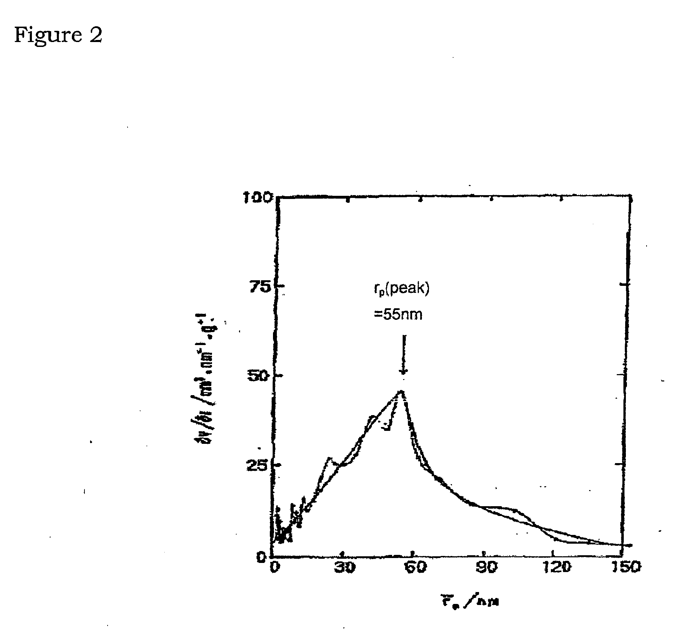

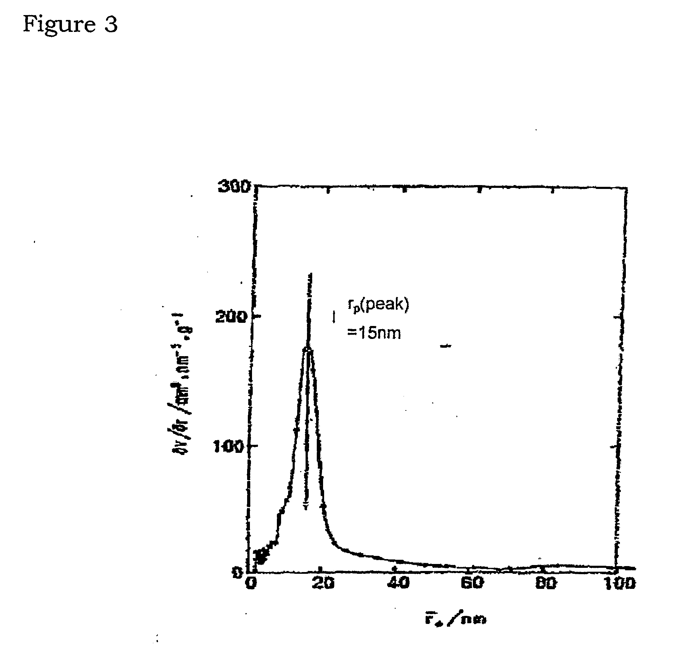

The benzene adsorption isotherm was obtained by the above measuring method, and the pore distribution curve was measured using the adsorbed side data on the basis of JIS-K1150, by the analyzing method by Dollimore Heal (D. Dollimore, G.R. Heal, J. Appl. Chem., 14.109 (1964)). In addition, as for a standard isotherm (benzene, 25..C) needed at the analyzing, the saturated steam pressure of benzene was confirmed at the analyzing by using the data described in Journal of Colloid and Interface Science 165, 532 - 535 (1994), and when it was changed, the saturated steam pressure was adjusted.
Measuring of the pore peak and pore peak radius: In the pore distribution curve, a part showing the maximum value of $\Delta Vp/\Delta logRp$ is determined as the pore peak, and the radius at the pore peak is determined as the pore peak radius and indicated as "nm". Examples are given in Figures 1 to 3.

Test example 2

Measuring method of oil absorption
The oil absorption was measured on the basis of JISK 6217-4 (the carbon black for rubber - basis characteristics). The oil absorption according to JISK 6217-4, relates to anhydrous, dried silica. However, in the present invention, the oil absorption relates to moist silica particles (including the loss on drying) obtained after the drying treatment for the commercial circulation was carried out. The intention is to know the properties of the actual usage form.
Furthermore, the index of the change of the oil absorption to the DBP dropping rate (the oil absorption Index 1 = OI1), and the influence of the oil absorption rate (the oil absorption Index 2 = OI2) were measured with the following formulas.

Oil absorption Index 1 (011): Total index of the change of the oil absorption to the DBP dropping rate

$$OI\,1\left[\frac{\min}{100g}\right]=\frac{OA\,[ml/100g]\,at\,DR=7[ml/\min]-OA\,[ml/100g]\,at\,DR=2[ml/\min]}{5[ml/\min]}\quad\text{Formula (I)}$$

DR = dropping rate; OA = Oil Absorption

When the OI1 value of the formula (I) is low, it can be said that the amorphous silica particles have a hard and strong structure and a high oil absorbing rate.

Oil absorption Index 2 (OI2)

$$OI\,2 = \frac{OA\,[ml/100g]\,at\,DR = 7[ml/\min] - OA\,[ml/100g]\,at\,DR = 4[ml/\min]}{OA\,[ml/100g]\,at\,DR = 4[ml/\min] - OA\,[ml/100g]\,at\,DR = 2[ml/\min]}\quad \text{Formula (II)}$$

DR = dropping rate; OA = Oil Absorption

In Table 1, the dropping rate, the OI 1, the difference of the oil absorption between the dropping rates of 4 ml/minute and 2 ml/minute ("A" in the following table), the difference of the oil absorption between the dropping rates of 7 ml/minute and 4 ml/minute ("B" in the following table), and the OI 2 of Examples 1, 2 and Comparison examples 1 to 3 were shown.

Table 1:

|  | Example 1 | Example 2 | Comparison example 1 | Comparison example 2 | Comparison example 3 |
|---|---|---|---|---|---|
| Pore peak radius (nm) | 42 | 55 | 15 | not clear | 12 |
| Oil absorption [ml/ 100g] when the dropping rate is 2 ml/minute | 295.0 | 330.0 | 294.3 | 283.1 | 273.1 |
| Oil absorption [ml/ 100g] when the dropping rate is 4 ml/minute | 318.1 | 352.0 | 304.0 | 312.0 | 283.1 |
| Oil absorption [ml/ 100g] when the dropping rate is 7 ml/minute | 334.3 | 359.3 | 318.1 | 333.1 | 298.7 |
| OI1 [min/100g] | 7.86 | 5.86 | 4.76 | 10.0 | 5.12 |
| A | 23.1 | 22.0 | 9.7 | 28.9 | 10.0 |
| B | 16.2 | 7.3 | 14.1 | 21.1 | 15.6 |
| OI2 | 0.70 | 0.30 | 1.45 | 0.73 | 1.56 |

OI1: The value was so low that the particles had a hard and high oil absorbing rate.
OI2: When the value was 1 or more, the oil absorbing rate was low after removing the structural factor. When the value was 1 or less, the oil absorbing rate was high after removing the structural factor.

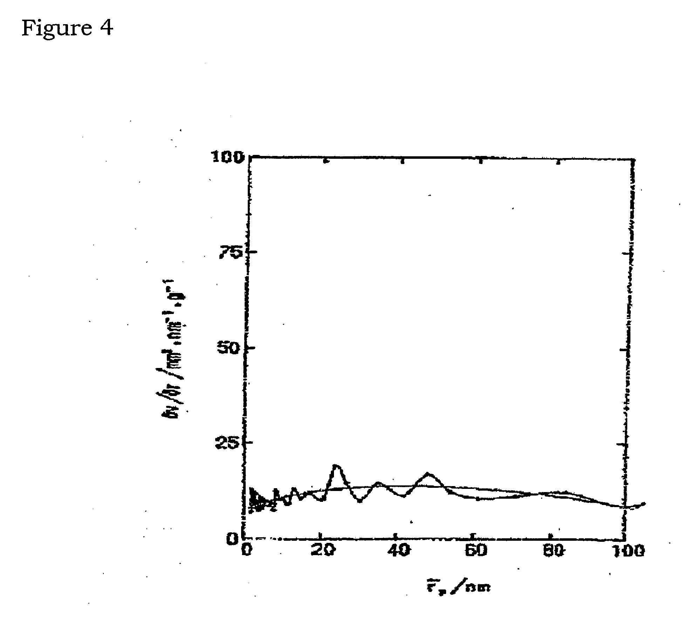

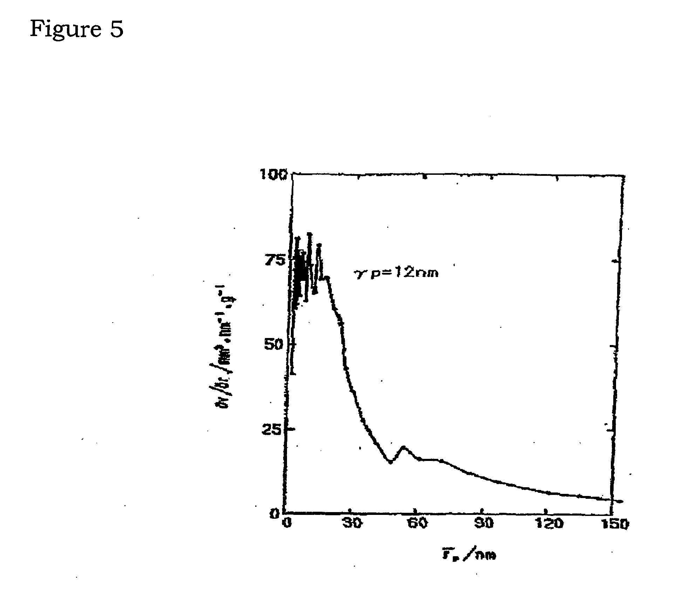

In the above results, the pore peak radius of the amorphous silica particles of Example 1 and 2 were from 30 nm or more to 90 nm or less. On the other hand, the pore peak radius of Comparison examples 1 to 3 were out of the above range. Further, the maximum value of $\Delta Vp/\Delta Rp$ was 30 mm$^3$/nm·g$^{-1}$ or more in Examples 1 and 2, as shown in Figures 1 to 5. On the other hand, as for the amorphous silica particles of Comparison example 2, the clear pore peak was not observed.

As for Comparison examples 1 and 3, the pore peak radius was small, and the value of OI1 calculated from the oil absorptions when the dropping rates were 2 mL/minute and 7 mL/minute was comparatively low. However, the value of OI2 being the index of the oil absorbing rate was 1 or more and the oil absorbing rate was low. That is, although the amorphous silica particles had hard structures, these had the low oil absorbing rates. On the other hand, as for Comparison example 2, it was considered that the particles did not have a specific pore peak radius, and had a high open structure. The value of OI1 was large, OI2 was 1 or less, and the structure was weak although the oil absorbing rate was high, and thus it was an easily collapsed amorphous silica particles.

As for Examples 1 and 2, the total indexes were the same as for Comparison examples 1 and 3, and index 2 was low as in Comparison example 2. Therefore, it was determined that these particles were the amorphous silica particles having hard and strong structures and high oil absorbing rates.

Test example 3

Specific surface area measuring method (Nitrogen adsorption method)

The specific surface area was measured by the nitrogen adsorption method, and as for the pore peak radius, the value calculated from the benzene adsorption method was used. More particularly, these were calculated by the following method.

The specific surface area was calculated by BET method, after measuring the adsorption isotherm of nitrogen gas of the sample being vacuum-degassed at 160 ˚C for 90 minutes by using the automatic specific surface area / pore distribution measuring apparatus BELSORP 28, which was made by Nippon Bel Co. Ltd. (References : S. Brunauer, P.H. Emmett, E.Teller, J. Amer. Chem. Soc., 60, 309 (1938))

Test example 4

Average particle size (volume average size) measuring method

It was measured by using a multisizer-II made by the Coulter Company, and a suitable aperture tube was selected.

Test example 5

Vitamin E oil absorption measuring method.

A pipe for dropping the vitamin E, where the hole diameter of the pipe was 1 mm, was mounted to BENCH KNEADER having a 2L total capacity (made by Irieshokai). Next, about 1L silica was filled in the kneader, and vitamin E where the viscosity was decreased by heating at about 60 to 80 ˚C was dropped to the silica to be oil absorbed while stirring. For pulverizing the lump generated at the oil absorption, after the oil absorption, the silica was stirred for 30 to 60 seconds with a juicing mixer.

Evaluation method : 2 to 5 g of the vitamin E absorbed particles were taken into a small type pulverizer made by Shibata Science Company (personal mill, SCM-40A type), and was stirred for about 30 seconds. The particle state was observed and the value of the oil absorption, which was just before the appearance of the particles changing from the fine powder to the fine particles or from white to yellow, was determined as the maximum oil absorption. Then, the oil absorption of vitamin E was calculated from the following formula.

Maximum oil absorption = Vitamin E absorption (g) / Silica weight before absorbing (g)

The measured results of the specific surface area, the average particle size and the vitamin E oil absorption were shown in Table 2.

Table 2:

|  | Example 1 | Example 2 | Comparison example 1 | Comparison example 2 | Comparison example 3 |
|---|---|---|---|---|---|
| Specific surface area (m$^2$/g) | 232 | 204 | 264 | 161 | 331 |
| Average particle size ($\mu$m) | 11.2 | 2.7 | 2.9 | 3.5 | 8.8 |
| Vitamin E absorption | 2.4 | 2.4 | 2.1 | - | 2.2 |

[0098] [0094] As for the amorphous silica particles, the specific surface area was 200 to 240 (m$^2$/g), and the average particle size was 2 to 12 $\mu$m. As for the vitamin E oil absorption, the vitamin E oil absorptions of the amorphous silica particles of Example 1 and 2 were higher than those in Comparison example 1 and 3, and thus it was found out that the amorphous silica particles of Example 1 and 2 were useful as the liquid adsorbent for pharmaceuticals, agrochemicals or the like.

Test example 6

Application of the amorphous silica particles as the matting agent for the coating material

Test of the matting effect of the coated film.

A two-pack polyurethane coating material (a drying type Retan PG80III Clear, made by Kansai Paint Co. Ltd.) was used as a coated film-forming agent. 4 parts of the sample were added to the two-pack polyurethane coating material to be dispersed at 2000 rpm for 15 minutes by using a disper disperser (AC magnetic stirrer type made by Yaskawa Electric Corporation: HXI-7). Then, a curing agent for coating polyurethane (a drying type Retan PG80III curing agent, made by Kansai Paint Co. Ltd.) was mixed (the ratio of the coating material to the curing agent is 90 to 10), and a diluent (toluene) was added to adjust the viscosity. Then, the sample was coated onto a black gloss paper using an applicator (a doctor blade 3mil, made by Ueshima Seisakusyo Co. Ltd.), and this paper was cured at 60 °C to prepare a coated film piece. Next, 60° Gloss (the glossiness (%)) was measured using a 300A gloss meter made by Nippondenshokukogyo Co. Ltd.

As for the amorphous silica particles of Example 1 and 2 and Comparison example 1, 60° mirror reflectance (%), which was the index of the matting effect ,was measured.

The measured 60° mirror reflectance (%) was shown in Table 3. As the result, it was cleared that the reflectance of Example 1 and 2 were low and the matting effects were also high, as compared with Comparison example 1.

Table 3:

|  | 60° mirror reflectance (%) |
| --- | --- |
| Example 1 | 10.7 |
| Example 2 | 4.2 |
| Comparison example 1 | 23 |

Test example 7:

Measuring method "Bulk density"
The instrument

1. Stainless steel sieve (Authorized JIS standard sieve) mesh width: 850micron, diameter:200mm
2. Sieve holder(stainless or plastic) side: 250mm, length: 250mm, high:150mm
3. Receiver(plastic) side: 330mm, length:270mm, depth: 10mm
4. measurement cup(transparency, plastic) capacity: 100 ± 1mL, bore: 50.0 ± 10.2mm, depth: 51.0 ± 0.2mm, thickness:5mm
5. Spatula (plastic) side: 120cm, length: 40mm, thickness: 5mm
6. Spatula (stainless) length :230mm

The receiver is set. Sieve holder is set upper the receiver. The sieve is set on the sieve holder. The measurement cup known weight is set in the middle of the receiver. The sample is transferred onto the sieve. The sample is dropped with spatula (stainless).(width:60-70mm, rate: two times per sec.) The sample is accumulated like conic in measurement cup. The level measurement cup of sample is weighted using a balance.
$$\text{Bulk density} = \frac{S}{100}$$

   S: sample weight
Brief description of the drawings
[Figure 1] A benzene adsorption isotherm of amorphous silica particles of Example 1, the pore peak radius: 42nm, Vp: 2625 mm$^3$ ·g$^{-1}$.
[Figure 2] A benzene adsorption isotherm of amorphous silica particles of Example 1, the pore peak radius:55

nm, Vp: 2578 mm$^3$ ·g$^{-1}$.

[Figure 3] A benzene adsorption isotherm of amorphous silica particles of Comparison example 1, the pore peak radius: 15nm, Vp: 2213 mm$^3$ ·g$^{-1}$.

[Figure 4] A benzene adsorption isotherm of amorphous silica particles of Comparison example 2, the pore peak radius: No peak, Vp: 1000 mm$^3$ ·g$^{-1}$.

[Figure 5] A benzene adsorption isotherm of amorphous silica particles of Comparison example 3, the pore peak radius: 12nm, Vp: 3312 mm$^3$ ·g$^{-1}$1.

**Claims**

1. Amorphous silica particles, wherein the maximum value of $\Delta$Vp/$\Delta$ Rp (where Vp is the pore volume [mm$^3$/g] and Rp is the pore radius [nm]) is 20 mm$^3$/nm·g$^{-1}$ or more in the pore distribution curve obtained by a benzene adsorption isotherm, and the pore peak radius when the $\Delta$Vp/$\Delta$Rp value is maximum is from 20 nm or more to 100 nm or less.

2. Amorphous silica particles according to Claim 1, wherein the maximum value of $\Delta$Vp/$\Delta$Rp (where Vp is the pore volume [mm$^3$/g] and Rp is the pore radius [nm]) is 30 mm$^3$/nm·g$^{-1}$ or more in the pore distribution curve obtained by a benzene adsorption isotherm, and the pore peak radius when the $\Delta$Vp/$\Delta$Rp value is maximum is from 30 nm or more to 90 nm or less.

3. Amorphous silica particles according to Claim 1 or 2, wherein the oil absorption measured by JISK6217-4 (a carbon black for rubber - basic characteristics) is more than 260 ml/ 100g.

4. Amorphous silica particles according to Claims 3, wherein the oil absorption measured by JISK6217-4 (a carbon black for rubber - basic characteristics) is more than 280 ml/100g.

5. Amorphous silica particles according to Claim 4, wherein the oil absorption measured by JISK6217-4 (a carbon black for rubber - basic characteristics) is more than 300 ml/100g.

6. Amorphous silica particles according to Claim 5, wherein the oil absorption measured by JISK6217-4 (a carbon black for rubber - basic characteristics) is more than 320 ml/ 100g.

7. Amorphous silica particles according to any one of Claims 1 to 6, wherein the OI1 is 9.5 or less.

8. Amorphous silica particles according to any one of Claims 1 to 7, wherein the OI2 is 1.2 or less.

9. Use of silica particles according to any one of Claims 1 to 8 as matting agent, adsorbent (carrier) for pharmaceuticals and/or agrochemicals, extender or filler of various rubbers.

10. An adsorbent for pharmaceuticals, agrochemicals, comprising the amorphous silica particles according to any one of Claim 1 to 8.

11. A matting agent, comprising the amorphous silica particles according to any one of Claim 1 to 8.

12. A process for preparing amorphous silica according to any one of claims 1 to 8, wherein at least one alkali metal silicate and at least one mineral acid are neutralized.

**Patentansprüche**

1. Amorphe Siliciumdioxidteilchen, bei denen der Maximalwert von $\Delta$Vp/$\Delta$Rp (worin Vp für das Porenvolumen [mm$^3$/g] und Rp für den Porenradius [nm] steht) in der mit Hilfe einer Benzoladsorptionsisotherme erhaltenen Porenverteilungskurve 20 mm$^3$/nm·g$^{-1}$ oder mehr beträgt und der Porenpeakradius am Maximum des $\Delta$Vp/$\Delta$Rp-Werts 20 nm oder mehr bis 100 nm oder weniger beträgt.

2. Amorphe Siliciumdioxidteilchen nach Anspruch 1, bei denen der Maximalwert von $\Delta$Vp/$\Delta$Rp (worin Vp für das Porenvolumen [mm$^3$/g] und Rp für den Porenradius [nm] steht) in der mit Hilfe einer Benzoladsorptionsisotherme erhaltenen Porenverteilungskurve 30 mm$^3$/nm·g$^{-1}$ oder mehr beträgt und der Porenpeakradius am Maximum des

$\Delta Vp/\Delta Rp$-Werts 30 nm oder mehr bis 90 nm oder weniger beträgt.

3. Amorphe Siliciumdioxidteilchen nach Anspruch 1 oder 2, bei denen die gemäß JISK6217-4 (Ruß für Kautschuk - grundlegende Eigenschaften) gemessene Ölabsorption mehr als 260 ml/100 g beträgt.

4. Amorphe Siliciumdioxidteilchen nach Anspruch 3, bei denen die gemäß JISK6217-4 (Ruß für Kautschuk - grundlegende Eigenschaften) gemessene Ölabsorption mehr als 280 ml/100 g beträgt.

5. Amorphe Siliciumdioxidteilchen nach Anspruch 4, bei denen die gemäß JISK6217-4 (Ruß für Kautschuk - grundlegende Eigenschaften) gemessene Ölabsorption mehr als 300 ml/100 g beträgt.

6. Amorphe Siliciumdioxidteilchen nach Anspruch 5, bei denen die gemäß JISK6217-4 (Ruß für Kautschuk - grundlegende Eigenschaften) gemessene Ölabsorption mehr als 320 ml/100 g beträgt.

7. Amorphe Siliciumdioxidteilchen nach einem der Ansprüche 1 bis 6, bei denen der OI1 9,5 oder weniger beträgt.

8. Amorphe Siliciumdioxidteilchen nach einem der Ansprüche 1 bis 7, bei denen der OI2 1,2 oder weniger beträgt.

9. Verwendung von Siliciumdioxidteilchen nach einem der Ansprüche 1 bis 8 als Mattierungsmittel, Adsorptionsmittel (Träger) für Pharmazeutika und/oder Agrochemikalien, Streckmittel oder Füllstoff für verschiedene Kautschuke.

10. Adsorptionsmittel für Pharmazeutika und Agrochemikalien, umfassend die amorphen Siliciumdioxidteilchen nach einem der Ansprüche 1 bis 8.

11. Mattierungsmittel, umfassend die amorphen Siliciumdioxidteilchen nach einem der Ansprüche 1 bis 8.

12. Verfahren zur Herstellung der amorphen Siliciumdioxidteilchen nach einem der Ansprüche 1 bis 8, bei dem man mindestens ein Alkalimetallsilicat und mindestens eine Mineralsäure neutralisiert.

**Revendications**

1. Particules de silice amorphe, dans lesquelles la valeur maximale de $\Delta Vp/\Delta Rp$ (où Vp représente le volume de pores [mm$^3$/g] et Rp le rayon des pores [nm]) est de 20 mm$^3$/nm.g$^{-1}$ ou plus dans la courbe de distribution des pores obtenue par un isotherme d'adsorption de benzène, et le rayon de pic des pores, lorsque la valeur $\Delta Vp/\Delta Rp$ est maximale, se situe dans la plage de 20 nm ou plus à 100 nm ou moins.

2. Particules de silice amorphe selon la revendication 1, dans lesquelles la valeur maximale de $\Delta Vp/\Delta Rp$ (où Vp représente le volume de pores [mm$^3$/g] et Rp le rayon de pores [nm]) est de 30 mm$^3$/nm.g$^{-1}$ ou plus dans la courbe de distribution des pores obtenue par un isotherme d'adsorption de benzène, et le rayon de pic des pores, lorsque la valeur $\Delta Vp/\Delta Rp$ est maximale, se situe dans la plage de 30 nm ou plus à 90 nm ou moins.

3. Particules de silice amorphe selon la revendication 1 ou 2, dans lesquelles l'absorption d'huile mesurée par la norme JISK6217-4 (un noir de carbone pour le caoutchouc - caractéristiques basiques) est supérieure à 260 ml/100 g.

4. Particules de silice amorphe selon la revendication 3, dans lesquelles l'absorption d'huile mesurée par la norme JISK6217-4 (un noir de carbone pour le caoutchouc - caractéristiques basiques) est supérieure à 280 ml/100 g.

5. Particules de silice amorphe selon la revendication 4, dans lesquelles l'absorption d'huile mesurée par la norme JISK6217-4 (un noir de carbone pour le caoutchouc - caractéristiques basiques) est supérieure à 300 ml/100 g.

6. Particules de silice amorphe selon la revendication 5, dans lesquelles l'absorption d'huile mesurée par la norme JISK6217-4 (un noir de carbone pour le caoutchouc - caractéristiques basiques) est supérieure à 320 ml/100 g.

7. Particules de silice amorphe selon l'une quelconque des revendications 1 à 6, dans lesquelles l'OI1 est de 9,5 ou moins.

8. Particules de silice amorphe selon l'une quelconque des revendications 1 à 7, dans lesquelles l'OI2 est de 1,2 ou moins.

9. Utilisation de particules de silice selon l'une quelconque des revendications 1 à 8 comme agent de couplage, adsorbant (véhicule) pour produits pharmaceutiques et/ou produits agrochimiques, allongeur ou charge de divers caoutchoucs.

10. Adsorbant pour produits pharmaceutiques ou produits agrochimiques, comprenant des particules de silice amorphe selon l'une quelconque des revendications 1 à 8.

11. Agent de couplage, comprenant les particules de silice amorphe selon l'une quelconque des revendications 1 à 8.

12. Procédé de préparation de silice amorphe selon l'une quelconque des revendications 1 à 8, dans lequel on neutralise au moins un silicate de métal alcalin et au moins un acide minéral.

Figure 1

Figure 2

Figure 3

Figure 4

Figure 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*